# FASCICULE DE BREVET EUROPEEN

(11) **EP 1 196 369 B1**
(45) Date de publication et mention de la délivrance du brevet: **17.03.2004**
(21) Numéro de dépôt: 00949565.6
(22) Date de dépôt: 28.06.2000
(51) Int. Cl.: C07C 51/42, C07C 51/47, C07C 55/14

(54) **PROCEDE DE FABRICATION D'ACIDE ADIPIQUE**
VERFAHREN ZUR HERSTELLUNG VON ADIPINSÄURE
METHOD FOR MAKING ADIPIC ACID

(30) Priorité: 29.06.1999 FR 9908591
(43) Date de publication de la demande: 17.04.2002
(73) Titulaire: Rhodia Polyamide Intermediates, 69192 Saint-Fons (FR)
(72) Inventeur: FELIX, Albert, F-69100 Villeurbanne (FR); ROQUES, Yves, F-69100 Villeurbanne (FR)
(74) Mandataire: Esson, Jean-Pierre
(86) Numéro de dépôt international: PCT/FR2000/001809
(87) Numéro de publication internationale: WO 2001/000557

(56) Documents cités:
- WO-A-98/35929
- FR-A- 1 208 145
- US-A- 3 123 635
- US-A- 3 886 153
- US-A- 5 471 001

## Description

La présente invention concerne un procédé de fabrication d'acide adipique, plus particulièrement de cristaux d'acide adipique.

Elle est relative plus concrètement à un procédé de traitement des cristaux d'acide adipique obtenus en sortie de cristallisation.

L'acide adipique est un grand produit intermédiaire, notamment dans le domaine des polymères et plus particulièrement du polyamide et dans la synthèse des polyuréthanes.

L'acide adipique est en général synthétisé par oxydation par l'acide nitrique d'un mélange cyclohexanone/cyclohexanol en présence de catalyseurs d'oxydation comme le vanadium et le cuivre.

L'acide adipique est récupéré et purifié par des opérations successives de cristallisation.

Au cours de ces opérations, l'acide adipique est notamment séparé des autres acides dicarboxyliques formés comme l'acide glutarique, l'acide succinique.

Les cristaux d'acide adipique produits en sortie de cristallisation sont généralement de forme oblongue et de surface très irrégulière.

L'acide adipique est généralement stocké dans des fûts ou containers de grandes dimensions et éventuellement transporté sur le lieu de son utilisation par exemple les installations de fabrication de polyamide ou de sel adipate d'hexaméthylène diamine.

Au cours de ce stockage et éventuellement transport, il se produit souvent un mottage, c'est-à-dire un collage de plusieurs cristaux entre eux. Ce phénomène est très pénalisant car il diminue fortement la coulabilité de l'acide adipique lors de l'alimentation des cristaux dans les installations par exemple de polymérisation.

Le brevet US 5 471 001 propose un procédé particulier de cristallisation de l'acide adipique avec utilisation d'ultra-sons. Les cristaux obtenus présentent une meilleure coulabilité et une faculté de "mottage" plus faible au cours du stockage et du transport.

Un des buts de la présente invention est de proposer un procédé permettant la fabrication de cristaux d'acide adipique présentant une forme et un état de surface particuliers pour obtenir une bonne coulabilité lors du chargement ou du déchargement, ou plus généralement du transport de ces cristaux, et une faculté de "mottage" diminuée.

A cet effet, l'invention propose un procédé de fabrication de cristaux d'acide adipique à partir d'acide adipique obtenu par cristallisation caractérisé en ce qu'il consiste à disperser les cristaux d'acide adipique recueillis en sortie de cristallisation dans un milieu liquide constitué par de l'eau ou un mélange eau/acide acétique avec une concentration pondérale en acide adipique supérieure ou égale à 5%, en agitant ledit milieu liquide puis à séparer lesdits cristaux dudit milieu liquide.

Les conditions de température pour la mise en oeuvre de ce traitement, et notamment pendant l'étape d'agitation du milieu liquide ne sont pas critiques.

Toutefois, il est préférable que dans le domaine de température choisi, la solubilité de l'acide adipique dans le milieu liquide reste faible, par exemple à une valeur inférieure à, environ, 2 g/l à 20°C. Le domaine de température préféré de l'invention est compris entre 20°C à 70°C, avantageusement entre 20°C et 60°C.

Dans certains cas, il pourra être avantageux de refroidir le milieu liquide à une température inférieure à 20°C, avant de séparer les cristaux dudit milieu liquide.

De même, la puissance de l'agitation devra être suffisante pour éviter des gradients de concentration trop importants en acide adipique ou autres composés contenus dans le milieu. Toutefois, cette puissance ne devra pas être trop élévé pour éviter de casser les cristaux.

Selon une nouvelle caractéristique de l'invention, la concentration pondérale en cristaux d'acide adipique dans le milieu liquide est supérieure à 5 % (rapport masse solide/ masse solide + masse liquide) et avantageusement entre 5 % et 60 % en poids.

La concentration en cristaux peut avoir une influence sur le résultat du traitement. En effet, plus le nombre de cristaux est important, plus l'effet de lissage de la surface de ceux-ci pourra être important. Toutefois, une concentration trop élevée peut être néfaste au procédé car elle ne permettra pas d'obtenir un effet de lisssage correct, et peut entraîner une agglomération de cristaux entre eux.

Selon l'invention, il est préférable que les cristaux d'acide adipique destinés à être dispersés présentent une taille moyenne comprise entre 100 µm et 1000 µm, avantageusement entre 200 et 700 µm.

Ces cristaux peuvent être soumis à un broyage préalable, si leur taille moyenne après cristallisation est trop élevée.

Après traitement selon le procédé, les cristaux ont une taille moyenne comprise entre 50 µm et 1000 µm environ.

Toutefois, les tailles moyennes précisées ci-dessus ne sont indiquées qu'à titre d'illustration et correspondent aux domaines préférentiels. Le procédé de l'invention peut également s'appliquer à des cristaux de taille moyenne inférieure ou supérieure.

Selon un mode de réalisation préféré de l'invention, le traitement des cristaux est obtenu par mise en mouvement du milieu liquide. Cette mise en mouvement du milieu liquide peut être une agitation de celui-ci réalisée par un ou plusieurs agitateurs présentant des formes de mobile variées et classiquement utilisées dans le domaine de l'agitation des suspensions.

Pour améliorer l'effet du traitement, des chicanes peuvent être disposées dans le réacteur contenant le milieu liquide.

Cette mise en mouvement du milieu liquide peut également être obtenue par une mise en rotation du milieu liquide dans un dispositif du type essoreuse ou centrifugeuse.

Enfin de manière générale, l'invention comprend tous les moyens et dispositifs capables de mettre en mouvement un liquide dans un réacteur ou une cuve. En effet, d'autres installations ou dispositifs que ceux décrits ci-dessus pourraient être utilisés sans sortir pour cela du cadre de l'invention.

Par ailleurs, le procédé de l'invention permet accessoirement de réaliser un lavage des cristaux d'acide adipique. Ainsi, la teneur en acide nitrique est fortement diminuée.

Les cristaux d'acide adipique traités selon le procédé de l'invention présente une forme de galet à surface lisse. Les galets ont des formes variées, notamment oblongues ne présentant aucune arête vive.

Les cristaux ainsi traités présentent une faible tendance au mottage. En outre, leur forme sans arête vive et leur surface lisse permettant d'obtenir un déplacement aisé d'un agglomérat par rapport à l'autre quand ils sont isolés du milieu liquide et séchés.

De ce fait, les cristaux d'acide adipique obtenus par le procédé de l'invention présentent une excellente coulabilité et une très faible faculté de mottage.

Il est donc possible de stocker et transporter ces produits pendant des durées longues et dans des conditions d'atmosphère non contrôlées.

Le remplissage des containers de stockage et de transport est aisé ainsi que le déstockage ou l'alimentation dans des réacteurs.

L'invention sera mieux illustrée au vu des exemples ci-dessous donnés uniquement à titre indicatif et en référence aux figures annexées dans lesquelles :
- la figure 1 représente une vue réalisée au microscope à balayage électronique avec un facteur de grossissement de 20, d'un échantillon de cristaux d'acide adipique non traité par le procédé de l'invention, et
- la figure 2 représente une vue réalisée au microscope à balayage élecronique avec un facteur de grossissement de 20 d'un échantillon des cristaux représentés à la figure 1 après traitement par le procédé de l'invention.

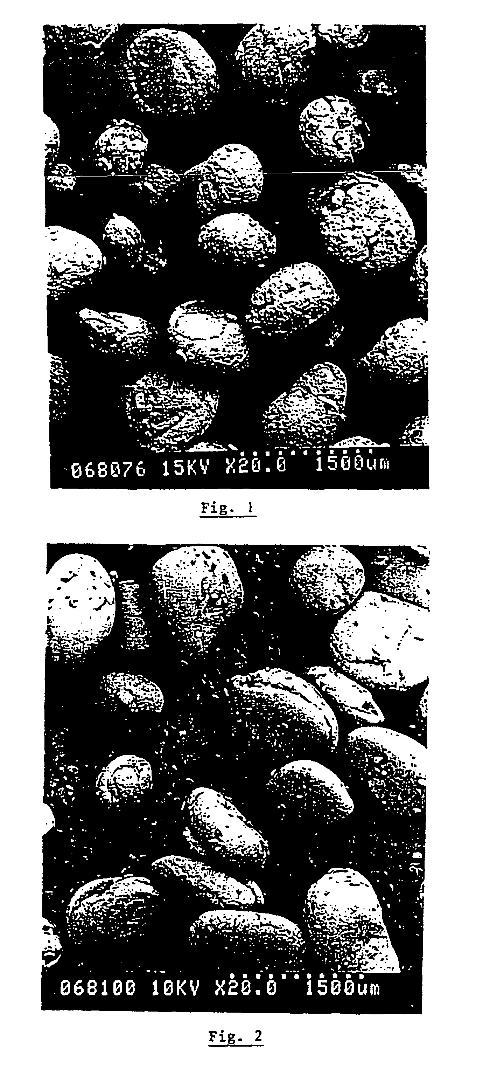

Des cristaux d'acide adipique obtenus par cristallisation à partir d'une solution aqueuse d'acide adipique ont une taille moyenne de 600 µm. La forme de ces cristaux est illustrée à la figure 1. Ces cristaux forment des blocs de forme oblongue présentant une surface très irrégulière comprenant des petits grains collés ou agglomérés en surface et de nombreuses fissures ou cavités. 200 g de cristaux sont dispersés dans 330 g d'eau contenue dans une cuve équipée d'un agitateur. La concentration des cristaux dans le milieu liquide est de 40 % en poids.

Le mélange est maintenu sous agitation pendant une heure à une température de 25°C.

Après filtration et séchage en lit fluidisé à 100°C pendant une heure, les cristaux d'acide adipique obtenus présentent une taille moyenne de 600 µm.

L'aspect de ces cristaux, illustré par la figure 2, démontre clairement l'effet du procédé de l'invention. En effet, les cristaux ont toujours une forme de galet oblongue, mais leur surface est lisse avec peu de particules collées.

Après un stockage des cristaux dans un emballage classique pendant plusieurs jours dans une atmosphère normale, leur alimentation dans un réacteur n'a posé aucun problème. Aucune agglomération ou mottage n'a été constaté.

## Revendications

1. Procédé de fabrication de cristaux d'acide adipique à partir d'acide adipique obtenu par cristallisation **caractérisé en ce qu'**il consiste à disperser les cristaux d'acide adipique recueillis en sortie de cristallisation dans un milieu liquide constitué par de l'eau ou un mélange eau/acide acétique avec une concentration pondérale en acide adipique supérieure ou égale à 5%, en agitant ledit milieu liquide puis à séparer lesdits cristaux dudit milieu liquide.

2. Procédé selon la revendication 1, **caractérisé en ce que** la température du milieu liquide est comprise entre 20°C et 70°C.

3. Procédé selon la revendication 1 ou 2, **caractérisé en ce que** la concentration pondérale en acide adipique dans le milieu liquide est comprise entre 5 % et 60 %.

4. Procédé selon l'une des revendications précédentes, **caractérisé en ce que** les cristaux d'acide adipique avant dispersion ont une taille moyenne comprise entre 100 µm et 1000 µm.

5. Procédé selon l'une des revendications précédentes, **caractérisé en ce que** les cristaux séparés de la dispersion ont une taille comprise entre 50 µm et 1000 µm.

6. Procédé selon l'une des revendications précédentes, **caractérisé en ce que** le milieu liquide est refroidi avant la séparation des cristaux traités.

## Claims

1. Process for the manufacture of adipic acid crystals from adipic acid obtained by crystallization, **characterized in that** it consists in dispersing the adipic acid crystals collected on conclusion of crystallization in a liquid medium composed of water or a water/acetic acid mixture with a concentration by weight of adipic acid of greater than or equal to 5%, in stirring the said liquid medium and in then separating the said crystals from the said liquid medium.

2. Process according to Claim 1, **characterized in that** the temperature of the liquid medium is between 20°C and 70°C.

3. Process according to Claim 1 or 2, **characterized in that** the concentration by weight of adipic acid in the liquid medium is between 5% and 60%.

4. Process according to one of the preceding claims, **characterized in that** the adipic acid crystals before dispersion have a mean size of between 100 µm and 1000 µm.

5. Process according to one of the preceding claims, **characterized in that** the crystals separated from the dispersion have a size of between 50 µm and 1000 µm.

6. Process according to one of the preceding claims, **characterized in that** the liquid medium is cooled before the separation of the treated crystals.

## Patentansprüche

1. Verfahren zur Herstellung von Adipinsäurekristallen, ausgehend von Adipinsäure, die durch Kristallisation erhalten wurde, **dadurch gekennzeichnet, daß** es darin besteht, die Adipinsäurekristalle, die nach einer Kristallisation gesammelt wurden, in einem flüssigen Medium, das aus Wasser oder einem Wasser/Essigsäure-Gemisch besteht, mit einer Gewichtskonzentration von Adipinsäure von 5 % oder darüber unter Rühren des flüssigen Mediums zu dispergieren und dann die Kristalle vom flüssigen Medium abzutrennen.

2. Verfahren nach Anspruch 1, **dadurch gekennzeichnet, daß** die Temperatur des flüssigen Mediums zwischen 20°C und 70°C liegt.

3. Verfahren nach Anspruch 1 oder 2, **dadurch gekennzeichnet, daß** die Gewichtskonzentration an Adipinsäure im flüssigen Medium zwischen 5 % und 60 % liegt.

4. Verfahren nach einem der vorangehenden Ansprüche, **dadurch gekennzeichnet, daß** die Adipinsäurekristalle vor einer Dispersion eine mittlere Größe zwischen 100 µm und 1000 µm haben.

5. Verfahren nach einem der vorangehenden Ansprüche, **dadurch gekennzeichnet, daß** die aus der Dispersion abgetrennten Kristalle eine Größe zwischen 50 µm und 1000 µm haben.

6. Verfahren nach einem der vorangehenden Ansprüche, **dadurch gekennzeichnet, daß** das flüssige Medium vor der Abtrennung der behandelten Kristalle gekühlt wird.
